# EUROPÄISCHE PATENTSCHRIFT

(11) **EP 0 423 580 B1**
(45) Veröffentlichungstag und Bekanntmachung des Hinweises auf die Patenterteilung: **03.01.1996**
(21) Anmeldenummer: 90119266.6
(22) Anmeldetag: 08.10.1990
(51) Int. Cl.: C07C 21/06

(54) **Verfahren zur Rückgewinnung von Vinylchlorid**
Process for the recovery of vinylchloride
Procédé pour la récupération de chlorure de vinyle

(30) Priorität: 19.10.1989 DE 3934796
(43) Veröffentlichungstag der Anmeldung: 24.04.1991
(73) Patentinhaber: BASF Aktiengesellschaft, D-67063 Ludwigshafen (DE)
(72) Erfinder: Deuschel, Walter, Dr., W-6700 Ludwigshafen 29 (DE); Lay, Peter, W-6700 Ludwigshafen (DE)

(56) Entgegenhaltungen:
- DE-A- 2 446 815
- CHEMICAL ABSTRACTS, Band 109, 26. Dezember 1988, Seite 326, Zusammenfassung Nr. 236090z, Columbus, Ohio, US; L.A. PAVLOVA et al.: "Removal of vinyl chloride from gases in the manufacture of PVC", & PLAST. MASSY 1988, (9), 50-1

## Beschreibung

Die vorliegende Erfindung betrifft ein verbessertes Verfahren zur Rückgewinnung von Vinylchlorid aus Vinylchlorid enthaltenden Gasen durch Absorption des Vinylchlorids in flüssigem, wasserhaltigem N-Methylpyrrolidon und anschließende thermische Desorption des Vinylchlorids aus dem wasserhaltigen N-Methylpyrrolidon.

Aus der DE-A 2 446 815 ist ein Verfahren zur Rückgewinnung und Reinigung von Vinylchlorid aus Polyvinylchloridreaktoren bekannt, nach dem das Vinylchlorid in wasserhaltigen N-Alkyllactamlösungen absorbiert wird und anschließend durch Destillation aus dem Lösungsmittel entfernt wird. Nachteilig an diesem Verfahren ist jedoch, daß eine Destillation der VC-haltigen Alkyllactamlösung zu einer weitgehend VC-freien Lösung einen hohen Energieaufwand erfordert.

Aufgabe der vorliegenden Erfindung war es, ein verbessertes Verfahren zur Rückgewinnung von Vinylchlorid aus N-Methylpyrrolidonlösungen zu entwickeln, das bei effektiver Entfernung des VC aus diesen Lösungen mit großer Wirtschaftlichkeit betrieben werden kann.

Demgemäß wurde ein verbessertes Verfahren zur Rückgewinnung von Vinylchlorid aus Vinylchlorid enthaltenden Gasen durch Absorption des Vinylchlorids in flüssigem, wasserhaltigem N-Methylpyrrolidon und anschließende thermische Desorption des Vinylchlorids aus dem wasserhaltigen N-Methylpyrrolidon gefunden, in dem
(a) das Vinylchlorid- und wasserhaltige N-Methylpyrrolidon aus einer Absorptionsanlage von 0,5 bis 5 Gew.-% Vinylchlorid und von 1 bis 20 Gew.-% Wasser enthält, und
(b) man das Vinylchlorid- und wasserhaltige N-Methylpyrrolidon mittels eines Wärmeaustauschers zunächst auf eine Temperatur im Bereich von 80 bis 120°C aufheizt und
(c) sodann die thermische Desorption des Vinylchlorids in einer ersten Stufe unter Bedingungen vornimmt, bei denen das desorbierte Vinylchlorid, das in einer Menge im Bereich von 30 bis 60 Gew.-% abgetrennt wird, bezogen auf die Gesamtmenge an Vinylchlorid, keine nennenswerten Mengen Wasser enthält, und
(d) anschließend in einer zweiten Desorptionsstufe das restliche Vinylchlorid zusammen mit Wasser austreibt, und
(e) das hierbei zwangsweise abgetrennte Wasser in die zweite Desorptionsstufe zurückführt, und
(f) die Vinylchlorid-freie heiße Sumpfphase der zweiten Desorptionsstufe in dem Wärmeaustauscher aus Schritt (b) im Gegenstrom mit dem aus der Absorptionsanlage stammenden Vinylchlorid- und wasserhaltigen N-Methylpyrrolidon abkühlt und zur Absorptionsanlage zurückleitet.

Die Vinylchlorid-haltigen wäßrigen NMP-Lösungen enthalten üblicherweise 0,5 bis 5 Gew.-% Vinylchlorid und 1 bis 20 Gew.-% Wasser. Die Absorption des VC in den NMP-Lösungen kann in bekannter Weise erfolgen. Eine vollständige VC-Austreibung ist jedoch notwendig, um in der Absorptionsstufe eine ausreichende Entfernung von VC zu erreichen.

Die Absorptionslösung wird zweckmäßigerweise zunächst auf 80 bis 120°C, vorzugsweise 100°C, mittels eines Wärmetauschers aufgeheizt und sodann in die erste Desorptionskolonne geleitet, wobei die Einleitung im allgemeinen im mittleren Drittel der Kolonne erfolgt. Die Betriebsbedingungen der Kolonne werden so gewählt, daß die ausgegasten Vinylchloriddämpfe keine nennenswerten Mengen Wasser enthalten. Üblicherweise wird die Kolonne bei Normaldruck und Temperaturen, die, je nach VC-Gehalt, im Bereich von 80 bis 120°C liegen, betrieben. Es ist auch möglich bei erhöhtem Druck (etwa bis zu 1,5 bar) oder unterhalb Normaldruck (etwa 0,5 bis 1 bar) zu arbeiten, wobei die Temperaturbereiche in dem Fachmann bekannter Weise entsprechend eingestellt werden können.

In dieser 1. Desorptionszone können 30 bis 60 Gew.-% gasförmiges Vinylchlorid aus der Absorptionslösung abgetrennt werden. Die VC-Dämpfe können über Kopf der Kolonne abgezogen werden. Aus dem Kolonnensumpf wird sodann eine Vinylchlorid abgereicherte N-Methylpyrrolidon-Lösung abgezogen und zur 2. Desorptionszone geleitet.

In dieser 2. Desorptionszone wird das restliche Vinylchlorid zusammen mit Wasser ausgetrieben, das aus der N-Methylpyrrolidon-Lösung stammt.

Die Einleitung erfolgt im allgemeinen in der Mitte einer Kolonne, die üblicherweise Füllkörper enthält und ca. 10 bis 30 theoretische Böden hat.

Die Kolonne wird üblicherweise bei Normaldruck betrieben. Die Sumpftemperatur kann 100 bis 200°C, vorzugsweise etwa 140°C, betragen. Am Kolonnenkopf kann das VC-Wasserdampf-Gemisch mit Temperaturen von 80 bis 110°C abgezogen werden. Dieses Gemisch kann dann zunächst mit dem aus der 1. Desorptionszone stammenden Vinylchlorid vereinigt und abgekühlt werden.

Anschließend wird das Wasser auskondensiert und zum Kolonnenkopf zurückgeleitet. Die abgetrennten Vinylchloridgase lassen sich beispielsweise in der PVC-Produktion wiederverwenden.

Aus dem Sumpf der 2. Desorptionszone kann eine flüssige NMP-Phase abgezogen werden, die aus 80 bis 99 Gew.-% NMP sowie 1 bis 20 Gew.-% Wasser besteht und keine nachweisbaren Mengen an Vinylchlorid enthält.

Zur Abkühlung wird die heiße Flüssigkeit in den Wärmeaustauscher geleitet, in dem im Gegenstrom die aus der Absorptionszone stammende VC-haltige NMP-Lösung erhitzt wird. Nach weiterer Abkühlung kann das VC-freie NMP-Wasser-Gemisch zur Wiederverwendung in die Absorptionszone geleitet werden.

Üblicherweise wird das Verfahren kontinuierlich ausgeführt.

Bevorzugt wendet man das erfindungsgemäße Verfahren auf Vinylchlorid enthaltende Abgase der Vinylchlorid-Herstellung oder der Vinylchlorid-Polymerisation an.

Das erfindungsgemäße Verfahren ermöglicht eine besonders wirtschaftliche Rückgewinnung von Vinylchlorid. Dadurch, daß im ersten Desorptionsschritt ein Großteil des Vinylchlorids ausgegast wird, läßt sich die Wasserdampf-Destillation im zweiten Desorptionsschritt mit geringerem Energieaufwand betreiben. Außerdem ist die VC-Entfernung aus der wäßrigen NMP-Lösung besonders effektiv (bis unter die Nachweisgrenze von VC), was sich bei der Wiederverwendung der Lösung im Absorptionsschritt positiv auf die Effektivität des Absorptionsvorgangs auswirkt.

### Beispiel

Pro Stunde wurden 2000 kg einer Lösung aus 93 Gew.-% N-Methylpyrrolidon, 6 Gew.-% Wasser und 1 Gew.-% Vinylchlorid, die bei der Absorption des Vinylchlorids aus Vinylchorid enthaltenden Gasen mittels wäßrigem NMP erhalten wurde, in den Mittelteil einer mit Füllkörpern gefüllten, bei etwa 150°C betriebenen Desorptionskolonne von 30 cm innerem Durchmesser und 450 cm Höhe gegeben. Am Kopf der Kolonne wurden stündlich 8 kg gasförmiges VC abgezogen. Der Rest des VC war im Sumpf enthalten.

Die Sumpfphase dieser ersten Desorptionskolonne wurde dann dem Mittelteil einer zweiten Desorptionskolonne zugeführt, die 25 cm lichte Weite und eine Höhe von 900 cm hatte. Die Füllkörperfüllung dieser Kolonne, die mit einem Rücklaufverhältnis von 8:1 betrieben wurde, entsprach etwa 12 theoretischen Böden. Die Sumpftemperatur betrug 140°C und die Kopftemperatur 100°C.

Das Wasserdampf-VC-Gemisch wurde am Kolonnenkopf abgezogen, mit dem VC-Dampf aus der 1. Desorptionsstufe vereinigt und in einen Kondensator geleitet, wo bei 35°C das Wasser abgeschieden wurde. Unter diesen Bedingungen fielen stündlich 20 kg VC an, die für die PVC-Produktion wiederverwendet wurden. Das abgeschiedene Wasser wurde zum Kolonnenkopf zurückgeleitet.

Die VC-freie heiße Sumpfphase der zweiten Kolonne (1980 kg/h) mit einem NMP-Gehalt von 94 Gew.-% wurde in einem Wärmeaustauscher im Gegenstrom der aus der Absorptionsanlage stammenden VC-haltigen NMP-Lösung auf 60°C abgekühlt und nach weiterer Kühlung auf 35°C zur Absorptionsanlage zurückgeleitet.

## Patentansprüche

1. Verfahren zur Rückgewinnung von Vinylchlorid aus Vinylchlorid enthaltenden Gasen durch Absorption des Vinylchlorids in flüssigem, wasserhaltigem N-Methylpyrrolidon und anschließende thermische Desorption des Vinylchlorids aus dem wasserhaltigen N-Methylpyrrolidon, dadurch gekennzeichnet, daß
(a) das Vinylchlorid- und wasserhaltige N-Methylpyrrolidon aus einer Absorptionsanlage von 0,5 bis 5 Gew.-% Vinylchlorid und von 1 bis 20 Gew.-% Wasser enthält, und
(b) man das Vinylchlorid- und wasserhaltige N-Methylpyrrolidon mittels eines Wärmeaustauschers zunächst auf eine Temperatur im Bereich von 80 bis 120°C aufheizt und
(c) sodann die thermische Desorption des Vinylchlorids in einer ersten Stufe unter Bedingungen vornimmt, bei denen das desorbierte Vinylchlorid, das in einer Menge im Bereich von 30 bis 60 Gew.-% abgetrennt wird, bezogen auf die Gesamtmenge an Vinylchlorid, keine nennenswerten Mengen Wasser enthält, und
(d) anschließend in einer zweiten Desorptionsstufe das restliche Vinylchlorid zusammen mit Wasser austreibt, und
(e) das hierbei zwangsweise abgetrennte Wasser in die zweite Desorptionsstufe zurückführt, und
(f) die Vinylchlorid-freie heiße Sumpfphase der zweiten Desorptionsstufe in dem Wärmeaustauscher aus Schritt (b) im Gegenstrom mit dem aus der Absorptionsanlage stammenden Vinylchlorid- und wasserhaltigen N-Methylpyrrolidon abkühlt und zur Absorptionsanlage zurückleitet.

2. Verfahren gemäß Anspruch 1, dadurch gekennzeichnet, daß man es auf Vinylchlorid enthaltende Abgase der Vinylchlorid-Herstellung oder der Vinylclorid-Polymerisation anwendet.

## Claims

1. A process for recovering vinyl chloride from vinyl chloride-containing gases by absorbing the vinyl chloride in liquid, water-containing N-methylpyrrolidone and subsequently thermally desorbing the vinyl chloride from the water-containing N-methylpyrrolidone, wherein
(a) the vinyl chloride- and water-containing N-methylpyrrolidone from an adsorption system contains from 0.5 to 5 % by weight vinyl chloride and from 1 to 20 % by weight water, and
(b) the vinyl chloride- and water-containing N-methylpyrrolidone is initially heated by a heat exchanger to from 80 to 120°C, and
(c) then the thermal desorption of the vinyl chloride is undertaken in a first stage under conditions at which the desorbed vinyl chloride, which is removed in an amount in the range from 30 to 60 % by weight based on the total amount of vinyl chloride, contains negligible amounts of water, and
(d) subsequently in a second desorption stage the remaining vinyl chloride is driven out together with water, and
(e) the water which is compulsorily removed in this way is returned to the second desorption stage, and
(f) the vinyl chloride-free, hot bottom phase from the second desorption stage is cooled in the heat exchanger from step (b) in countercurrent to the vinyl chloride- and water-containing N-methylpyrrolidone derived from the absorption system, and is passed back to the absorption system.

2. A process as claimed in claim 1, which is applied to vinyl chloride-containing off-gases from the preparation or polymerization of vinyl chloride.

## Revendications

1. Procédé de récupération de chlorure de vinyle dans des gaz contenant du chlorure de vinyle, par absorption du chlorure de vinyle dans de la N-méthylpyrrolidone hydratée liquide, suivie de désorption thermique du chlorure de vinyle à partir de la N-méthylpyrrolidone hydratée, caractérisé en ce que
a) la N-méthylpyrrolidone contenant du chlorure de vinyle et de l'eau provenant d'une installation d'absorption, contient de 0,5 à 5% en poids de chlorure de vinyle et de 1 a 20% en poids d'eau, et
b) on commence par chauffer la N-méthylpyrrolidone contenant du chlorure de vinyle et de l'eau à une température dans la gamme de 80 à 120°C, au moyen d'un échangeur de chaleur,
c) puis on procède a la désorption thermique du chlorure de vinyle, dans un premier étage, dans des conditions dans lesquelles le chlorure de vinyle désorbé, qui est séparé dans une proportion comprise entre 30 et 60% en poids par rapport au poids total de chlorure de vinyle, ne contient pas de quantités appréciables d'eau.
d) on extrait ensuite, dans un second étage de désorption, le chlorure de vinyle restant en même temps que de l'eau,
e) on renvoie dans le second étage de désorption l'eau ainsi séparée par force, et
f) on refroidit la phase de bas de colonne chaude du second étage de désorption, débarrassée du chlorure de vinyle, dans l'échangeur de chaleur de l'étage (b) à contre-courant de la N-méthylpyrrolidone contenant du chlorure de vinyle et de l'eau et provenant de l'installation d'absorption, et on la dirige en retour vers l'installation d'absorption.

2. Procédé selon la revendication 1, caractérisé en ce qu'on l'applique aux gaz résiduaires contenant du chlorure de vinyle, provenant de la préparation de chlorure de vinyle ou de la polymérisation de chlorure de vinyle.
